# EUROPÄISCHE PATENTANMELDUNG

(11) **EP 2 095 814 A1**
(43) Veröffentlichungstag der Anmeldung: **02.09.2009**
(21) Anmeldenummer: 08075144.9
(22) Anmeldetag: 26.02.2008
(51) Int. Cl.: A61K 9/20, A61K 45/06, A61P 25/34

(54) **Medikamentengestützter Nikotinentzug**

(71) Anmelder: Kox, Wolfgang J., 14129 Berlin (DE); Hensel, Mario, 13189 Berlin (DE)
(72) Erfinder: Kox, Wolfgang J., 14129 Berlin (DE); Hensel, Mario, 13189 Berlin (DE)
(74) Vertreter: Boeckh, Tobias

(57) **Zusammenfassung**

Die Erfindung betrifft die Verwendung von anticholinerg und antinoradrenerg wirksamen Substanzen zur Behandlung des Nikotinentzugssyndroms. Die Erfindung führt zur Blockade einer überschüssigen Acetylcholin- bzw. Noradrenalinfreisetzung in der initialen Phase des Nikotinentzuges und damit zu einer Reduktion der Entzugssymptomatik.

## Beschreibung

Die Erfindung betrifft die Verwendung von anticholinerg und antinoradrenerg wirksamen Substanzen zur Behandlung des Nikotinentzugssyndroms. Die Erfindung führt zur Blockade einer überschüssigen Acetylcholin- bzw. Noradrenalinfreisetzung in der initialen Phase des Nikotinentzuges und damit zu einer Reduktion der Entzugssymptomatik.

Die Gefährlichkeit der im Tabakrauch enthaltenen Giftstoffe für die Gesundheit ist wissenschaftlich unbestritten. Tabakrauch beinhaltet mehr als 400 Inhaltsstoffe, von diesen sind über 50 als potenzielle Kanzerogene bekannt. Passivrauchen ist in hohem Maße krebserregend und hat Herz-Kreislauf Erkrankungen zur Folge. Die Zahl der Toten durch Passivrauchen wird für Deutschland auf jährlich mindestens 3 300 geschätzt. Passivrauch ist vermutlich der quantitativ bedeutsamste inhalative Krankheitsauslöser in der Innenraumluft (Radon, Nowak, "Passivrauchen - aktueller Stand des Wissens", Deutsche Medizinische Wochenschrift 2004, 157-162).

Nikotin, ein wesentlicher Bestandteil des Tabaks, ist eine der am schnellsten süchtig machenden Substanzen. Es hat nicht nur psychostimulierende Wirkungen wie Kokain oder Amphetamine, sondern es beeinflusst im Gehirn eine Vielzahl von Neuromodulatoren (1, 2). Nikotin greift v.a. an den nikotinergen Acetylcholinrezeptoren des zentralen Nervensystems an. Infolge der Bindung an den Rezeptor kommt es zu sehr komplexen neuronalen Interaktionen und zur Ausschüttung der verschiedensten Neurotransmitter wie Dopamin, Serotonin, Noradrenalin und Endorphine (1, 2, 3, 4). Die nikotinergen Acetylcholinrezeptoren haben einen sehr engen Bezug zum präfrontalen Cortex, wodurch bestimmte Hirnfunktionen wie Aufmerksamkeit, Gedächtnis und Lernen durch Nikotin verbessert werden können (1, 5, 6). Außerdem besteht eine enge räumliche Beziehung zum dopaminergen Belohnungssystem (ventrale Area tegmentalis, Limbisches System, Amygdala und Nucleus acumbens), einer entwicklungsgeschichtlich entscheidenden Struktur (1, 4).

Neben dem Nikotin enthalten Zigaretten eine ganze Reihe von Substanzen, die sich in ihrer Suchtwirkung potenzieren. So verstärkt beispielsweise Ammonium, welches dem Tabak bei der Verarbeitung künstlich zugesetzt wird, die Nikotinwirkung. Während des Verbrennungsprozesses entsteht u.a. Acetaldehyd. Dieser Stoff bewirkt eine Reduzierung des Enzyms Monoaminooxidase B (MAO-B). Durch dieses Enzym werden im Gehirn Neurotransmitter wie Dopamin und Serotonin abgebaut. Es konnte gezeigt werden, dass Raucher bis zu 40% weniger MAO-B haben als Nichtraucher (5). Dementsprechend mehr Dopamin und Serotonin wirkt auf das Gehirn ein, was das Suchtpotential letztlich erhöht. Im Entzug steigt die MAO-Aktivität wieder an. Dieser Effekt kann durch Atropin vermindert werden (6).

Die plötzliche Abstinenz vom Nikotin kann bei starken Rauchern zu erheblichen Entzugserscheinungen führen. Dazu gehören Craving (Suchtdruck), Unruhe, Konzentrationsstörungen, Aggressivität, Angstzustände, depressive Verstimmungen, Schlafstörungen, Bluthochdruck, Schwitzen und gastrointestinale Probleme. Die Ausprägung dieser Symptome kann individuell sehr unterschiedlich sein und hängt von einer Reihe von Faktoren ab (Dauer und Intensität des Nikotinabusus, Lebensalter, Einstiegsalter, Geschlecht u.s.w.). In der Regel treten diese Erscheinungen in den ersten 14 Tagen nach Abstinenzbeginn auf, wobei die Intensität stetig abnimmt. Allerdings können einige Symptome, im Sinne eines protrahierten Abstinenzsyndroms, noch über Monate beobachtet werden.

Laut Drogenbericht der Bundesregierung (www.droqenbeauftragte.de) versuchen 35 Prozent der Raucher durchschnittlich fünfmal pro Jahr mit dem Rauchen aufzuhören. Davon sind jedoch nur 4 - 5 Prozent nach einem Jahr noch abstinent. Auf der Suche nach Unterstützung sieht sich der aufhörwillige Raucher einer Vielzahl von Behandlungsangeboten gegenüber. Allerdings muss festgestellt werden, dass die Behandlungsergebnisse bislang eher unbefriedigend sind, sowohl im Hinblick auf die Zahl der Raucher, die erfolgreich den Zigarettenkonsum eingestellt haben, als auch bezüglich der erzielten Langzeitabstinenz (Tabelle 1).

**Tabelle 1. Wirksamkeit von Rauchentwöhnungsmethoden**

| Methode | Erfolgsquote |
|---|---|
| Placebobehandlungen | < 10% |
| Hypnose/ Akupunktur | < 15% |
| Selbsthilfe | < 10% |
| Nikotinersatz | 15-25% |
| Bupropion | 30% |
| Bupropion + Nikotinersatz | 35% |
| Verhaltenstherapie (Gruppe) | 20-30% |
| Verhaltenstherapie + Nikotinersatz | 25-35% |

In den letzten Jahren sind einige medikamentöse Entzugstherapien auf den Markt gelangt oder in der Entwicklung, deren Vor- und Nachteile hier kurz beschrieben werden sollen:

Das in Pillenform unter dem Handelsnamen Zyban verabreichte Medikament enthält den Wirkstoff Bupropion-Hydrochlorid SR (Bupropion). Dieser Wirkstoff wird schon seit Beginn der 90er Jahre in den USA unter dem Produktnamen Wellbutrin gegen Depressionen eingesetzt. Bupropion-Hydrochlorid (Zyban) wirkt als selektiver Inhibitor der Dopamin- und Noradrenalinwiederaufnahme im Zentralnervensystem. Die Substanz wurde primär als Antidepressivum entwickelt, findet aber in den letzten Jahren zunehmende Anwendung als "Anti-Raucher-Mittel", d.h. der Wirkstoff von Zyban greift direkt im Gehirn dort ein, wo die Abhängigkeit entsteht - im Dopamin-System. Der Wirkstoff gehört zur SSRI-Gruppe (Serotonin-Wiederaufnahme-Hemmer). Wie Nikotin lässt auch Zyban die Menge des Botenmoleküls Dopamin in den Zwischenräumen der Nervenzellen, den Synapsen, in die Höhe schnellen. Dadurch werden die Entzugserscheinungen quasi "gepuffert". Nach einem Bericht des "New England Journal of Medicine" (7) schafften etwa ein Drittel der Raucher den Ausstieg aus ihrer Sucht mit Zyban. Neben den bisherigen Erfolgsmeldungen mehren sich in der letzten Zeit Berichte, die auf gravierende Probleme im Umgang mit Zyban hinweisen. Erste Warnungen von Experten der Arzneimittelkommission werden laut. In Großbritannien werden bereits 57 Todesfälle mit Zyban in Verbindung gebracht. In Deutschland und anderen Ländern ist das Mittel mithin umstritten und wird als fragwürdig angesehen.

Auf einen anderen Wirkmechanismus als bisherige Präparate gegen Nikotinabhängigkeit baut Rimonabant. Es greift in das so genannte Endocannabinoid-System ein, das im Gehirn Suchtgefühle reguliert. Rezeptoren dieses Systems sind auch auf der Oberfläche von Fettzellen zu finden. Dadurch wird nicht nur das Verlangen nach Nahrung oder Nikotin im Gehirn reduziert, der Stoff hemmt auch die Einlagerung von neuen Fettzellen. Rimonabant wurde jedoch die arzneimittelrechtliche Zulassung verweigert, auch weil es mit einem erhöhten Suizidrisiko in Verbindung gebracht wird.

Bekannt ist ferner die so genannte Detox-N-Methode, bei der auf der Basis eines medikamentengestützten Nikotinentzugsverfahrens, welches bereits 1986 von Bachynsky beschrieben wurde (8), ein multimodales Behandlungskonzept entwickelt wurde, welches es den Rauchern erlaubt, möglichst schnell und frei von Entzugssymptomen abstinent zu werden.

Neben den Imbalancen des cholinergen Transmittersystems kommt es während des Nikotinentzuges v.a. zu Ungleichgewichten des noradrenergen Systems. In diesem Zusammenhang hat sich die Anwendung des Alpha-2-Agonisten Clonidin als erfolgversprechende Therapieoption erwiesen. Clonidin vermindert in bestimmten Hirnstrukturen, wie dem Locus coeruleus, die Noradrenalinfreisetzung. Glassman und Mitarbeiter setzten in einer randomisierten, plazebokontrollierten Studie Clonidin bei Vorliegen einer schweren Nikotinabhängigkeit (> 60 Zigaretten pro Tag) ein und fanden in der Therapiegruppe einen um 50% höheren Behandlungserfolg (Abstinenz), als in der Kontrollgruppe (9). Gourlay et al. konnten in einer Dosisfindungsstudie zeigen, dass mit steigender Clonidindosierung auch der Anti-Craving-Effekt (suchtdruckmindernde Wirkung) zunahm (10). Außerdem gelang der selben Autorengruppe in einer Meta-Analyse der Nachweis, dass Clonidinmedikation die Rauchentwöhnung erleichtert (11). Darüber hinaus gibt es Hinweise aus Tierversuchen, dass Clonidin nicht nur einen Einfluss auf die Noradrenalin- sondern auch auf die Acetylcholin-Freisetzung hat (12).

Insofern beschreibt die EP 1 408 968 B1 die kombinierte Anwendung anticholinerg und antinoradrenerg wirksamer Substanzen zur Behandlung des Nikotin-Entzugssyndroms (NES). Gemäß der EP 1 408 968 B1 ist eine Wirkstoffkombination von Atropin, Scopolamin und Clonidin zum Nikotinentzug bevorzugt, wobei eine derartige Wirkstoffkombination in Form eines Arzneimittels als Vakzine oder auch als Nasenspray verabreicht werden kann. Weiterhin beschreibt die Druckschrift ein Hautpflaster, umfassend Kontaktflächen mit den genannten Substanzen.

Nachteilig bei der beschriebenen Methode ist es, dass die Patienten mit unangenehmen körperlichen Reaktionen konfrontiert werden wie Schmerzen bei der Injektion sowie Akkomodationsstörungen, Schwindel und Mundtrockenheit. Derartige Nachteile reduzieren die Erfolge einer nachhaltigen Rauchentwöhnung und führen zu unerwünschten Rückfällen der Patienten.

Ziel der vorliegenden Erfindung ist es daher, die genannten Nachteile zu vermeiden und eine alternative Darreichungsform zur Verfügung zu stellen, die zu einer bleibenden Rauchentwöhnung beiträgt.

Gelöst wird die Aufgabe durch die Merkmale der unabhängigen Ansprüche.

Der medikamentengestützte Nikotinentzug gemäß der vorliegenden Erfindung ist auf die kombinierte Anwendung anticholinerger und antinoradrenerger Substanzen zur Behandlung des Nikotin-Entzugssyndroms gerichtet. Diese pharmakologische Intervention soll zur Blockade einer überschüssigen Acetylcholin- bzw. Noradrenalinfreisetzung in der initialen Phase des Nikotinentzuges und damit zu einer Reduktion der Entzugssymptomatik führen.

Erfindungsgemäß ist mithin die Verwendung einer Wirkstoffkombination aus Atropin, Scopolamin sowie eines Sedativums für eine intramuskulös zu verabreichende Zusammensetzung zur therapeutischen Behandlung des Nikotinentzugs vorgesehen.

Als besonders bevorzugt erweist sich die Verwendung eines Sedativums, da dieses zum einen dazu führt, die vorübergehenden Begleiterscheinungen der Anticholinergika wie Mundtrockenheit, leichter Schwindel und Akkomodationsstörungen zu kupieren. Andererseits hat der Patient durch die resultierende Schlafphase das Gefühl einer Zäsur (switch on - switch off effect). Bei dem Sedativum kann es sich um eines der nachfolgend genannten Substanzen handeln: Chlorpromazin, Promethazin, Promazin, Triflupromazin, Levomepromazine oder Methotrimeprazine sowie Derivate und Salze der vorgenannten Substanzen. Besonders bevorzugt ist Levopromazinhydrogenmaleat, welches in einer therapeutischen Breite von 20 bis 50 mg zum Einsatz kommt, besonders bevorzugt 25 mg.

Erfindungsgemäß ist die Verwendung an Atropin mit einer therapeutischen Breite zwischen 0,1 bis 0,5, an Scopolamin bei 0,1 bis 0,5 mg und des Sedativums zwischen 25 und 75 mg vorgesehen.

Besonders bevorzugt ist eine wirksame Menge an Atropin bei 0,2 mg, an Scopolamin bei 0,2 mg und des Sedativums zwischen 25 und 75 mg.

Die erfindungsgemäße Wirkstoffkombination wird als "single Dosis" verabreicht, d.h. die Mengenangaben der beanspruchten Bestandteile beziehen sich jeweils auf eine einzige Applikation. Die im Rahmen der vorliegenden Erfindung genannten Mengenangaben entsprechen den wirksamen Mengen eines als "normal" eingestuften Patienten mit einem Körpergewicht von ca. 70 bis 90 kg. Bei hiervon abweichenden Parametern können die Mengenangaben innerhalb der therapeutischen Breite reduziert bzw. erhöht werden.

Besonders bevorzugt ist mithin die Verwendung einer Wirkstoffkombination aus Atropin, Scopolamin sowie eines Sedativums zur Herstellung eines Arzneimittels für eine intramuskulär zu verabreichende Zusammensetzung zur therapeutischen Behandlung des Nikotinentzugs, wobei das Arzneimittel zur Verabreichung in einer Einzeldosis von Atropin zwischen 0,1 bis 0,5 mg an wirksamer Menge, von Scopolamin zwischen 0,1 bis 0,5 mg an wirksamer Menge und des Sedativums zwischen 25 und75 mg an wirksamer Menge hergerichtet ist.

Um den Injektionsschmerz zu minimieren, der nicht nur aus dem Setzen einer Spritze resultiert, sondern auch durch das Injizieren der erfindungsgemäß verwendeten Substanzen (Atropin, Scopolamin), wird entweder der Wirkstoffkombination selbst ein Lokalanästhetikum zugesetzt oder aber es wird dasselbe lokal an der Einspritzstelle appliziert. Bevorzugt wird Lidocain als Lokalanästhetikum verwendet.

Um den Nikotinentzug zu erleichtern und zu unterstützen wird erfindungsgemäß die beschriebene medikamentöse Behandlung mittels Clonidin ergänzt, wobei Clonidin in einer wirksamen Menge von 0,1 bis 0,3 mg/Tag, bevorzugt 2,1 mg/Tag oral verabreicht, ggf. über mehrere Tage hinweg, dann in geringerer Dosis, nämlich 0,075 bis 0,225 mg/Tag, bevorzugt 0,15 mg/Tag.

Demgemäß ist Gegenstand der Erfindung auch die Verwendung von Clonidin zur Herstellung eines Arzneimittel zur Begleitung einer therapeutischen Behandlung des Nikotinentzugs, wobei dieses Arzneimittel zur Verabreichung in einer täglichen Dosis von 0,1 bis 0,3 mg hergerichtet ist.

Ein weiterer Aspekt der Erfindung ist die alternative Verwendung eines Scopolaminpflasters. Hierbei handelt es sich um ein sogenanntes transdermales (Membran)Pflaster, wobei der Wirkstoff Scopolamin zeitlich verzögert, d.h. verlangsamt freigesetzt wird (Retard-Effekt) und mithin nur langsam vom Körper resorbiert wird. Derartige Pflaster sind grundsätzlich bekannt, beispielsweise als Mittel gegen Reisekrankheit und Übelkeit, jedoch nicht in Zusammenhang mit der vorliegenden Erfindung zum Nikotinentzug und in Wirkstoffkombination. Demgemäß ist Gegenstand der Erfindung auch die Verwendung einer Wirkstoffkombination aus Atropin sowie eines Sedativums zur Herstellung eines Arzneimittels für eine intramuskulär zu verabreichende Zusammensetzung zur therapeutischen Behandlung des Nikotinentzugs, wobei das Arzneimittel zur Verabreichung unter gleichzeitiger Applikation eines transdermalen Hautpflasters, enthaltend eine therapeutisch wirksame Menge an Scopolamin, hergerichtet ist.

Der Vorteil der Verwendung eines transdermalen Hautpflasters liegt unter anderem darin, dass nur wenige Hersteller Scopolamin überhaupt noch produzieren und mithin eine Alternative zu der 3-Wirkstoffkombination geschaffen wird, die wie beschrieben einem Patienten injiziert werden kann.

Die Erfindung sieht weiterhin ein Ampullenset und einen Kit vor. Dieses Ampullenset dient der Lagerung der separaten Komponenten der Wirkstoffkombination, also Atropin, Scopolamin sowie eines Sedativums. Die Ampullen bestehen vorzugsweise aus lichtundurchlässigem Material, zumindest aber aus einem Material, welches den in der jeweiligen Ampulle befindlichen Wirkstoff vor Lichteinflüssen schützt. Es hat sich gezeigt, dass eine unmittelbar vor der Injektion stattfindende Vermengung der Wirkstoffe Atropin und Scopolamin die besten Resultate bringt, da ein Gemisch aus Atropin/Scopolamin nur begrenzt haltbar ist. Die biologische Halbwertszeit liegt bei ca. 8 Tagen.

Es ist daher auch vorgesehen, dass die Ampullen mittels eines Adapters bzw. Verbindungsstückes miteinander verbindbar ausgestaltet sind, so dass die jeweiligen Wirkstoffe miteinander vermengt werden können und sodann mittels einer Injektionsspritze aus den Ampullen bzw. aus der Ampulle, in der sich das Gemisch befindet, aufgezogen und somit entnommen werden können.

Besonders bevorzugt ist auch ein dementsprechend ausgestalteter Kit, der die einzelnen Bestandteile enthält, also ein Ampullenset mit den Wirkstoffen Atropin und Scopolamin, sowie ein Sedativum. Ferner enthält der Kit einen Adapter, der korrespondierend zu den Bemaßungen der Ampulle(n) ausgestaltet ist und der Vermengung der in den Ampullen befindlichen Substanzen dient. Daneben ist eine Bedienungsanleitung bzw. ein Beipackzettel vorgesehen, der die einzelnen Bestandteile des Kits erläutert und ggf. auch die Vermischung der Substanzen und die richtige Applikation beschreibt.

Im übrigen kann der Kit auch die für eine Maßnahme des Nikotinentzugs notwendige Menge an Clonidin enthalten, so dass dem jeweiligen Arzt mit einem solchen Kit alle notwendigen Wirkstoffe, Substanzen und Hilfsmittel zur Verfügung gestellt werden, die er für die Anwendung benötigt.

Weitere vorteilhafte Maßnahmen sind in den übrigen Ansprüchen beschreiben. Die Erfindung wird anhand eines Ausführungsbeispiels und Figuren nunmehr näher beschrieben:
- Figur 1 zeigt: das Abstinenzverhalten als Abstinenzrate von 652 Rauchern nach 12 Monaten (in %)
- Figur 2 zeigt: den Abstinenzverlauf

Nach einer Reihe von Untersuchungen (Blutdruck, Herzfrequenz, EKG, Auskultation von Herz und Lunge, Lungenfunktionsprüfung) werden 0,004 mg Atropinsulfat und 0,004 mg Scopolaminhydrobromid sowie 0,5-1,0 mg Chlorpromazin intramuskulär injiziert. Darauf folgt eine 30-minütige Überwachungsphase. Sind die Kreislaufverhältnisse in dieser Zeit stabil, wird der Patient bei einsetzender Sedativawirkung in Begleitung nach Hause entlassen. Die weitere Medikation besteht in der oralen Anwendung von 0,003 mg Clonidin, verteilt auf 2-3 Tagesdosen (über einen Zeitraum von 14 Tagen).

Die klinische Wirksamkeit hat sich bei nahezu 3000 durchgeführten Therapien nachweisen lassen. In einer an 670 Rauchern durchgeführten Langzeitbeobachtung konnte die Abstinenzrate und die möglichen Nebenwirkungen ermittelt werden. Hierzu eine kurze Beschreibung der Therapie und der Ergebnisse nach der Detox-N-Methode:

Bei der beschriebenen Medikation handelt es sich um einen "off label use" (Anwendung zugelassener Arzneimittel, wobei sich die Zulassung nicht auf die beabsichtigte Indikation richtet). Die Outcome-Daten (Abstinenz bzw. Rückfälligkeit, Entzugserscheinungen, Rückfallgründe, Nebenwirkungen, Gesamturteil über die Therapie) wurden in strukturierten Telefoninterviews erhoben. Die Intensität der Entzugssymptomatik wurde mit Hilfe der Minnesota Nicotine Withdrawal Scale eruiert. In der vorliegenden Untersuchung wurden alle Patienten als abstinent eingestuft, die 12 Monate nach Beendigung der Pharmakotherapie rauchfrei waren. Als Therapieversager wurden jene Fälle gewertet, die während der Medikamenteneinnahme, also während der ersten 14 Tage, rückfällig wurden.

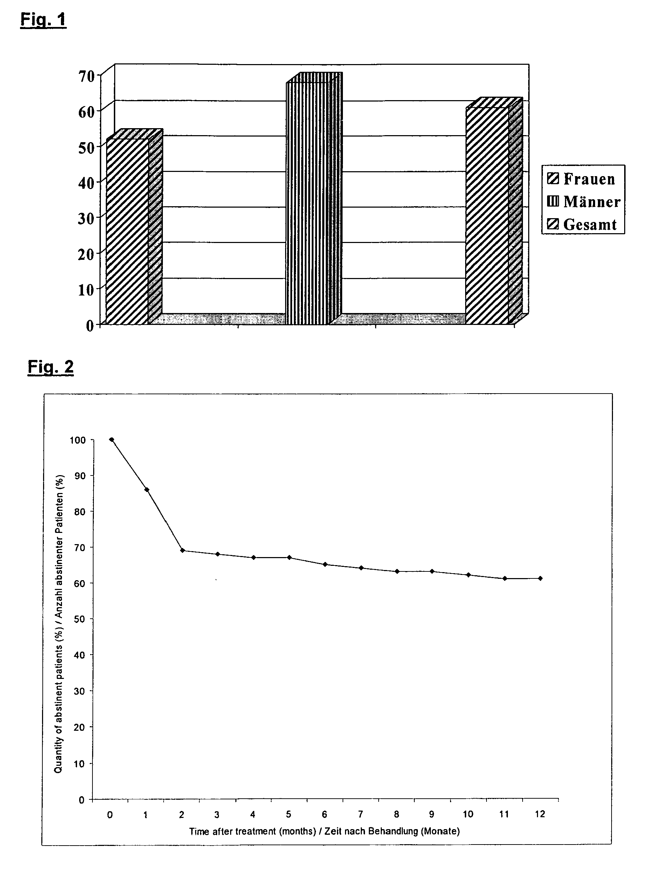

Von 652 Rauchern (280 Frauen, 372 Männer), über die Outcome-Daten erhoben werden konnten, lebten nach einem Zeitraum von mindestens 12 Monaten 399 (61%) abstinent und 253 (39%) waren rückfällig (Figur 1). Deutliche Unterschiede ergaben sich zwischen den Geschlechtern. Während 68% der Männer rauchfrei lebten, gelang dies nur 52% der Frauen. Weitere Determinanten für den Abstinenzerfolg waren der Schweregrad der körperlichen Abhängigkeit und der tägliche Zigarettenkonsum. So waren Raucher mit einer sehr starken bzw. starken körperlichen Abhängigkeit (Abstinenzrate 66%) erfolgreicher, als jene mit mittlerer, geringer oder sehr geringer Abhängigkeit. Die Gruppe der erfolgreich therapierten Raucher konsumierte im Vorfeld der Behandlung im Durchschnitt 38 (± 19) Zigaretten pro Tag während die rückfälligen Raucher einen Zigarettenkonsum von 30 (± 14) Zigaretten täglich angaben. Von den rückfälligen Rauchern brachen 39 die Behandlung bereits während der ersten 14 Tage ab und mussten als Therapieversager eingestuft werden (6%). Ansonsten ereignete sich die Mehrzahl aller Rückfälle (81 %) innerhalb der ersten 6 Wochen nach Ende der medikamentösen Therapie (Figur 2). Als häufigste Rückfallgründe wurden extreme Stress-Situationen, Unachtsamkeit, Gewichtszunahme, Obstipation und Craving (Suchtdruck) angegeben. Insgesamt konnte die Entzugssymptomatik bei den meisten Patienten effektiv unterdrückt werden. So wiesen 74% der Patienten einen Wert < 5 auf der Entzugssymptomskala auf (Minimalwert: 0, Maximalwert: 28). Einen Punktwert > 10 erreichten lediglich 7 Patienten.

Gravierende Nebenwirkungen im Sinne gefährdeter Vitalfunktionen wurden in keinem Fall beobachtet. Achtzehn Prozent der Patienten berichteten über Mundtrockenheit, 8% über Müdigkeit und 6% beklagten ein leichtes Schwindelgefühl, welches besonders innerhalb der ersten 2 Tage auftrat.

Diese Ergebnisse belegen die überraschende und außerordentlich erfolgreiche Behandlung von Rauchern zur Erlangung einer Tabakabstinenz.

### Referenzliste

[1] Dani JA, Heinemann S. Molecular and cellular aspects of nicotine abuse. Neuron 16 (1996) 905-908.
[2] Gamberino WC, Gold MS. Neurobiology of tobacco smoking and other addictive disorders. Psychiatr Clin North Am 22 (1999) 301-312.
[3] Wessler I, Kirkpatrick Ch J. Konsequenzen für den Nikotinkonsum durch das ubiquitäre Zytomolekül Acetylcholin. Deutsches Ärzteblatt 97 (2000) B41-B42.
[4] Levin ED, Simon BB. Nicotine acetylcholine involvement in cognitive function in animals. Psychopharmacology-Berl 138 (1998) 217-230.
[5] Berlin 1, Anthenelli RM. Monoamine oxidases and tobacco smoking. Int J Neuropsychopharmacol 4 (2001) 33-42.
[6] Mohammed YS, Mahfouz MM. Inhibition of rat brain and heart monoamine oxidase by atropine. Biochem Pharmacol 26 (1977) 871-874.
[7] Jorenby et a/. A controlled trial of sustained release bupropion, a nicotine patch, or both for smoking cessation. New England Journal of Medicine 340 (1999) 685-691.
[8] Bachynsky N. The use of anticholinergic drugs for smoking cessation: a pilot study. Int J Addict 21 (1986) 789-805.
[9] Glassman P. Heavy smokers, smoking cessation, and clonidine. JAMA 259 (1988) 2863-2866.
[10] Gourlay W. A placebo-controlled study of three clonidine doses for smoking cessation. Clin Pharmacol Ther 55 (1994) 64-69.
[11] Gourlay SG, Stead LF, Benowitz NL. Clonidine for smoking cessation. Cochrane Database Syst Rev 2 (2000) CD000058.
[12] Abelson KS, Hoglund AU. The effects of the alpha2-adrenergic receptor agonists clonidine and rilmenidine, and antagonists yohimbine and efaroxan, on the spinal cholinergic receptor system in the rat. Basic Clin Pharmacol Toxicol 94 (2004) 153-160.

## Patentansprüche

1. Verwendung einer Wirkstoffkombination aus Atropin, Scopolamin sowie eines Sedativums zur Herstellung eines Arzneimittels für eine intramuskulär zu verabreichende Zusammensetzung zur therapeutischen Behandlung des Nikotinentzugs.

2. Verwendung nach Anspruch 1, wobei die wirksame Menge an Atropin zwischen 0,1 bis 0,5 mg, an Scopolamin bei 0,1 bis 0,5 mg und des Sedativums zwischen 25 und75 mg liegt.

3. Verwendung nach Anspruch 2 wobei die wirksame Menge an Atropin bei 0,2 mg, an Scopolamin bei 0,2 mg und des Sedativums zwischen 25 bis75 mg liegt.

4. Verwendung einer Wirkstoffkombination aus Atropin, Scopolamin sowie eines Sedativums zur Herstellung eines Arzneimittels für eine intramuskulär zu verabreichende Zusammensetzung zur therapeutischen Behandlung des Nikotinentzugs, wobei das Arzneimittel zur Verabreichung in einer Einzeldosis von Atropin zwischen 0,1 bis 0,5 mg an wirksamer Menge, von Scopolamin zwischen 0,1 bis 0,5 mg an wirksamer Menge und des Sedativums zwischen 25 und75 mg an wirksamer Menge hergerichtet ist.

5. Verwendung einer Wirkstoffkombination aus Atropin sowie eines Sedativums zur Herstellung eines Arzneimittels für eine intramuskulär zu verabreichende Zusammensetzung zur therapeutischen Behandlung des Nikotinentzugs, wobei das Arzneimittel zur Verabreichung unter gleichzeitiger Applikation eines transdermalen Hautpflasters, enthaltend eine therapeutisch wirksame Menge an Scopolamin, hergerichtet ist.

6. Verwendung nach Anspruch 5, wobei die wirksame Menge an Atropin zwischen 0,1 bis 0,5 mg und des Sedativums zwischen 25 und75 mg liegt.

7. Verwendung nach Anspruch 5 oder 6, wobei die wirksame Menge an Atropin bei 0,2 mg und des Sedativums zwischen 25 bis75 mg liegt.

8. Verwendung nach wenigstens einem der Ansprüche 1 bis 7, wobei das Sedativum ausgewählt ist aus der Gruppe folgender Substanzen: Chlorpromazin, Promethazin, Promazin, Triflupromazin, Levomepromazine oder Methotrimeprazine sowie Derivate und Salze der vorgenannten Substanzen.

9. Verwendung nach Anspruch 8 wobei es sich bei dem Sedativum um Levopromazinhydrogenmaleat handelt.

10. Verwendung nach Anspruch 9, wobei die wirksame Menge des Levopromazinhydrogenmaleats bei 25 und bis 50 mg liegt.

11. Verwendung nach Anspruch 10, wobei die wirksame Menge des Levopromazinhydrogenmaleats bei 25 mg liegt.

12. Verwendung nach einem oder mehreren der Ansprüche 1 bis 11, wobei die intramuskulär zu verabreichende Wirkstoffkombination durch eine einmalige Injektion verabreicht wird.

13. Verwendung nach einem oder mehreren der Ansprüche 1 bis 12, wobei ein Lokalanästhetikum der Wirkstoffkombination beigesetzt ist.

14. Verwendung nach einem oder mehreren der Ansprüche 1 bis 12, wobei ein Lokalanästhetikum lokal an der Einstichstelle appliziert wird.

15. Verwendung nach den Ansprüchen 13 oder 14, **dadurch gekennzeichnet, dass** es sich bei dem Lokalanästhetikum um Lidocain handelt.

16. Verwendung nach Anspruch 5, wobei das Hautpflaster eine über einen Zeitraum von mehreren Tagen gleichmäßig verlangsamt freisetzende wirksame Menge an Scopolamin von 75 bis 1,5 mg aufweist.

17. Verwendung von Clonidin zur Herstellung eines Arzneimittel zur Begleitung einer therapeutischen Behandlung des Nikotinentzugs nach den Ansprüchen 1 bis 16, wobei dieses Arzneimittel zur Verabreichung in einer täglichen Dosis von 0,1 bis 0,3 mg hergerichtet ist.

18. Verwendung nach Anspruch 17, wobei die tägliche Dosis an Clonidin 0,075 bis 0,225 mg, bevorzugt 0,15 mg, beträgt.

19. Verwendung nach Anspruch 17 oder 18, wobei das Arzneimittel in einer oral zu verabreichenden Tabletten- oder Kapselform vorliegt.

20. Ampullenset zur Lagerung der separaten Komponenten der Wirkstoffkombination nach den Anspruch 1, 4 oder 5.

21. Ampullenset nach Anspruch 20, wobei die Wirkstoffe in den Ampullen durch das Ampullenmaterial lichtgeschützt sind.

22. Ampullenset nach Anspruch 20 oder 21, wobei die Ampullen über einen Adapter miteinander derart verbindbar sind, dass die in den Ampullen befindlichen Wirkstoffe miteinander vermengt werden können.

23. Ampullenset nach einem oder mehreren der Ansprüche 20 bis 22, wobei die einzelnen Wirkstoffe oder die miteinander vermengten Wirkstoffe über eine Injektionsnadel der jeweiligen Ampulle entnehmbar sind.

24. Kit, zumindest bestehend aus einem Ampullenset nach einem oder mehrere der Ansprüche 20 bis 23, sowie einer Bedienungsanleitung und/oder Beipackzettels.

25. Kit nach Anspruch 24, umfassend ferner
a. einen Adapter und eine Injektionsspritze mit Nadel, und/oder
b. das Sedativum in einer zur Anwendung geeigneten Form und Menge, und/oder
c. ein Lokalanästhetikum in einer zur Anwendung geeigneten Form und Menge, und/oder
d. das Arzneimittel Clonidin in einer zur oralen Applikation geeigneten Form und Menge.

26. Kit nach Anspruch 24 bis 26, wobei die einzelnen Bestandteile des Kits, insbesondere die jeweiligen Wirkstoffe und Arzneimittel in einer für eine einzige Nikotinentzugsmaßnahme erforderlichen Menge bereitgestellt und hergerichtet sind.
